(19) **Europäisches Patentamt / European Patent Office / Office européen des brevets**

(11) **EP 3 142 547 B1**

(12) **EUROPEAN PATENT SPECIFICATION**

(45) Date of publication and mention
of the grant of the patent:
**21.03.2018 Bulletin 2018/12**

(21) Application number: **15717591.0**

(22) Date of filing: **31.03.2015**

(51) Int Cl.:
*A61B 5/0205* (2006.01)  *G06F 19/00* (2018.01)
*A61B 5/00* (2006.01)

(86) International application number:
**PCT/GB2015/050993**

(87) International publication number:
**WO 2015/173539 (19.11.2015 Gazette 2015/46)**

(54) **METHOD AND APPARATUS FOR MONITORING PATIENT STATUS**

VERFAHREN UND VORRICHTUNG ZUR ÜBERWACHUNG DES PATIENTENZUSTANDES

PROCÉDÉ ET APPAREIL DE SURVEILLANCE DU STATUT D'UN PATIENT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.05.2014 GB 201408469**

(43) Date of publication of application:
**22.03.2017 Bulletin 2017/12**

(73) Proprietor: **OBS MEDICAL LIMITED
Abingdon, Oxfordshire OX14 4SD (GB)**

(72) Inventors:
• **STRACHAN, Iain Guy David
Abingdon
Oxfordshire OX14 4SD (GB)**
• **MOHSENI, Hamid Reza
Abingdon
Oxfordshire OX14 4SD (GB)**

(74) Representative: **J A Kemp
14 South Square
Gray's Inn
London WC1R 5JJ (GB)**

(56) References cited:
**WO-A1-2012/160350**      **US-A1- 2008 154 513**
**US-A1- 2011 068 929**      **US-A1- 2012 095 520**
**US-B2- 7 031 857**

• **David A Clifton ET AL: "Patient-Specific
Biomedical Condition Monitoring in
Post-operative Cancer Patients", The sixth
International Conference on Condition
Monitoring and Machinery Failure Prevention
Technology, 1 January 2009 (2009-01-01), pages
424-433, XP055192562, Ireland Retrieved from the
Internet:
URL:http://www.robots.ox.ac.uk/~davidc/pub
s/cancerhosp.pdf [retrieved on 2015-06-01]**
• **ZHANG Y ET AL: "Patient-specific learning in real
time for adaptive monitoring in critical care",
JOURNAL OF BIOMEDICAL INFORMATICS,
ACADEMIC PRESS, NEW YORK, NY, US, vol. 41,
no. 3, 1 June 2008 (2008-06-01), pages 452-460,
XP022700528, ISSN: 1532-0464, DOI:
10.1016/J.JBI.2008.03.011 [retrieved on
2008-03-28]**

## Description

The present invention relates to a method and apparatus for monitoring the status of a patient as measured by a plurality of vital signs.

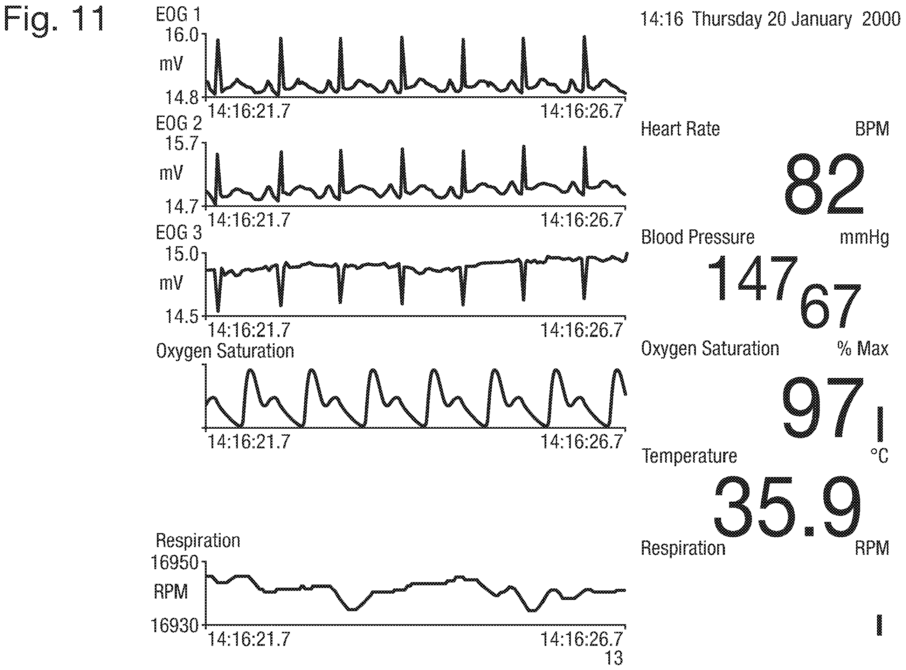

It is common in a clinical setting, particularly a high dependency care unit or intensive care unit, to monitor continuously the condition or status of patients. Typically vital signs such as one or more of blood pressure, heart rate, skin or body temperature, blood oxygen saturation (for instance measured by pulse oximetry with a finger probe), breathing rate (for instance measured by electrical impedance pneumography) may be monitored. The heart rate, and sometimes breathing rate, may be obtained from one or more channels of an electrocardiogram (ECG), which may also be monitored continuously. It is also possible to derive from these primary signals some secondary parameters such as heart rate variability, S-T segment elevation/depression and so on. Normally the individual measurements are displayed to a clinician in a form of a number, and possibly a graphical trace, as illustrated, for example, in Figure 11. However over recent years techniques for combining these individual parameters into a score which gives some overall indication of the patient's condition or status have been in use. To date, though, the majority of such scoring regimes in patient monitoring systems tend to be "one size fits all", in that the same scoring rules are applied irrespective of the subject. This is true of the modified early warning score (MEWS system), explained, for example in "The value of modified early warning score (MEWS) in surgical in-patients: a prospective observational study" by J Gardner-Thorpe, N Love, J Wrightson, S Walsh and N Keeling, (Ann. R. Coll. Surg. Engl. October 2006; 88(6): PP571-575). It is also true of the present applicant's own Visensia system explained in US-B2-7031857 which uses an FDA-cleared population-based statistical model of patient condition against which current measurements are compared.

However, normal values for vital signs can vary considerably from one patient to another due to factors such as age, type of disease, medication, type of surgery, etc. Replacing the population-based statistical model with a personal model for an individual patient is problematic because no valid set of measurements for developing such a model exists for an individual, especially when they have just changed status, for example because of a surgical operation or developing a disease or other medical condition. In the applicant's own US-B2-7031857 it was proposed that after an initial period of judging a patient's condition against a population-based statistical model, enough measurements on the individual patient would have been obtained to replace the population based model with a patient-specific one. However, in practice this is not possible as a patient's condition is changing and obtaining regulatory clearance for monitoring on the basis of a not-yet validated set of data is impossible. Therefore it is difficult to provide a scoring system which can provide a reliable and valid indication of variations from normality for an individual patient. Further it would require a long period of monitoring the patient once they have reached a stable state to provide a statistically reliable set of measurements from which a valid model can be generated. At the start of monitoring therefore, when the patient may be most at risk of sudden deterioration, a personalised model is not available. So it is not clear how to obtain a personalised model which is useful in a reasonably quick time.

US-A1-2012/0095520 discloses a method and system for discriminating cardiac events as treatable or untreatable by comparing then first to a population-based threshold, and if they cannot be classified on that basis, it then establishes a patient-specific threshold and compares the cardiac event to that. US-A1-2008/0154513 discloses a method and system for diabetes monitoring by comparing glucose levels to absolute levels based inter alia on population data and an individual threshold determined via analysis of the glycaemic patterns of each individual. US-A1-2011/0068929 discloses a patient condition alarm which judges parameters against two thresholds, one which may be arbitrary or reflect the result of a measurement on a patient, and a second which is parameter and/or patient-group specific. The paper "Patient-Specific Biomedical Condition Monitoring in Post-operative Cancer Patients"; David A. Clifton et al., The sixth International Conference on Condition Monitoring and Machinery Failure Prevention Technology, (2009), pp424-433 proposes replacing population-generic models of normality with patient-specific ones. The paper: "Patient-specific learning in real time for adaptive monitoring in critical care"; Y Zhang and P Szolovitz; Journal of Biomedical Informatics, Academic Press, New York, NY, US, Vol. 41, No. 3, June 2008, pp 452-460 discusses the idea of on-line learning of a patient-specific model. Another prior art method according to the preamble of claim 1 is known from WO 2012/160350 A1. The present invention provides an enhancement to a continuous patient monitoring system such as that disclosed in US-B2-7031857, on which the precharacterizing portion of claim 1 is based, by comparing the status of a patient, as measured by a plurality of vital signs, simultaneously to two different statistical models, one being population-based and one being personal to the patient. The statistical model which is personal to the patient is obtained by applying machine learning techniques to an initial population-based model and updating it based on each current vital signs measurement from the patient as it is received. By simultaneously evaluating the patient's status against two statistical models an alert notification can be generated relative to the population-based model if a computed score representing the patient's condition breaches a predefined threshold (this model can be a regulatory approved model), thus providing safety for the patient if their condition deteriorates, but also a notification of a change in the patient's status compared to the patient-specific statistical model can also be generated. Such a change in status could be an improvement or deterioration for that particular patient, but which is still within an overall region of normality for the population, and it can thus provide an earlier indication of change.

In more detail, therefore, the present invention provides a method of monitoring the status of a patient as defined by claim 1.

The first threshold on the first numerical index in the population-based reference model effectively defines a global region of normality for the population. The region of parameter space occupied by the patient's current state would normally be expected to be smaller than the global region of normality and may be embedded within it. The second threshold effectively defines a region of normality personal to the individual patient and changes from this could reflect either an improvement or deterioration in the individual patient's condition depending on whether the change is accompanied by an increase or decrease in the first numerical index obtained by reference to the population-based model.

The vital signs measured may typically comprise heart rate, breathing rate, blood pressure, blood oxygen saturation and body temperature and at each time point the measured values form a multi-parameter vital signs observation for evaluation against the statistical models. Typically the various parameters are collected at different sampling rates, for example the heart rate may be calculated every few seconds whereas the blood pressure may be measured only once every 2 to 4 hours, and so to provide a continuous set of samples the lower sampling rate measurements are repeated in successive multi-parameter observations until they are re-measured.

The selective enabling of the updating step when the patient status is within the global normality region for the population, i.e. when the first numerical index does not exceed the first threshold avoids updating the model based on noisy or spurious measurements. The inhibition on updating can be automatic based on the first numerical index, or can be under the control of a clinician. The threshold defining the region of global normality and the threshold for determining whether or not to update the patient specific model with an individual measurement can be different from each other. Thus it is possible for the clinician to selectively allow the patient-specific model to be updated even if the population-based model shows some degree of abnormality. In the case of one or more of the parameters in an observation being spurious or missing it is possible for this to be treated as a missing variable (for example replaced by its mean value) in determining whether to update the patient-specific model, but if it is decided to update then that the complete original observation is used to update the model. The decision on whether to update may be automatic, e.g. based on the degree of abnormality, or under the control of the clinician or operator.

The step of updating the patient-specific model can be stopped when the model becomes stable (for example when the parameters do not change significantly for a predetermined period of time, e.g. 20 or 30 minutes), and restarted when a comparison of the most recent multi-parameter vital signs observation (or some predefined number of them) with the patient-specific model indicates a change in patient status.

Preferably the patient-specific model is initialised to be identical to the population model, or to have the same statistical properties (e.g. mean and variance) as the population model such that it would give approximately the same values for the numerical indices. As patient-specific observations are received, this initialized model evolves to cover the area of normality for the particular patient being monitored.

The patient- specific model and the population-based model may be Kernel Density Estimators, such as a plurality of spherical Gaussian density functions with all kernels having equal weights. The patient's specific model may be updated by a Bayesian process which comprises calculating from the patient-specific model the likelihood of the most recent multi-parameter vital signs observation, multiplying the model's prior probability distribution by the calculated likelihood and then renormalizing it to form the Bayesian posterior probability distribution for the model, given the current sample, which then serves as the prior probability distribution for the next sample.

This update process for the patient-specific model may utilise particle filtering. This is a standard technique in which "particles" (being samples initially generated from the population-based model by random sampling of it) have a noise term added to each of them and then the likelihood of each particle plus noise is computed. A plurality of the particles is then resampled with a probability of selection being set proportional to its computed likelihood. The resampled set of particles constitutes the new patient-specific model. As an alternative to the standard particle filtering approach, the new samples can be drawn by sampling from the nearest kernel (to the selected particle) in the population-based model. This has the effect that the patient-specific model is always bounded by the population-based model. If it is desired for certain patients that a patient specific model may lie outside the population-based region of normality, then the standard resampling approach should be used.

As mentioned above the status change notification generated with reference to the patient-specific model can correspond to an improvement or deterioration in the patient's condition and it thus provides extra information to clinical staff compared to the results from the population-based reference model. Preferably an alert based on the patient-specific model is only generated if the current observation or observations are associated with an increase in the first numerical index generated with reference to the population-based reference model. This means that an alert is only generated if it reflects deterioration in the patient's condition.

Preferably such an increase of the first numerical index is judged by comparing the value of the first numerical index obtained for the current multi-parameter vital signs observation to the value of the first numerical index that was obtained at some previous time, for example at the time when the patient-specific model became stable or when the monitoring process was started.

To avoid generating alerts or status change notifications when spurious or erroneous measurements are received (for example because of noisy signals), the notifications may only be generated if a threshold is exceeded by a filtered version of the index (e.g. for more than a predefined number of observations in succession or more than a predefined number of observations within a predetermined time period, though other filtered versions may be used). This effectively smoothes the output of the system.

The invention may be embodied in an apparatus for executing the method and such an apparatus may therefore comprise an input for receiving the patient's vital signs measurements, a memory for storing the statistical models, a processor programmed to execute the steps of comparing the measurement to the statistical models, calculating the numerical indices and comparing them to the thresholds, and to output the alert and status change notifications. The output may comprise a display for displaying the notifications in a graphical form, though audible notifications may also be generated. Typically the display can display the individual measured vital signs in a conventional manner together with the numerical indices and alert and status notifications.

The apparatus may be incorporated into a standard vital signs monitor or can be embodied in a programmed computer system which receives the vital signs measurements.

The invention will be further described by way of examples with reference the accompanying drawings in which:-

Figure 1 conceptually illustrates regions of normality in statistical models for a population-based model and a patient-specific model;

Figure 2 schematically illustrates the operation of a system according to an embodiment of the present invention;

Figure 3(a) shows a time series of the population-based numerical index and Figures 3 (b) to (f) show a time series of five vital signs for a patient;

Figure 4 illustrates the first two principal components of the global population model in one embodiment of the invention;

Figure 5 illustrates the first two principal components of the centre of kernels for the population model together with the centre of Gaussian kernels for the patient-specific model in one embodiment of the invention;

Figure 6 illustrates the numerical indices calculated from population-based, patient-specific and combined models of Figure 5;

Figure 7(a) to (f) show the population-based index (a) and the five vital signs (b) to (f) for a patient with a large artefactual observation;

Figure 8 illustrates the corresponding first two principal components for the measurements of Figure 7;

Figure 9 illustrates the first two components for the measurements of Figure 7 against the patient-specific model of Figure 5;

Figure 10 schematically illustrates a system for executing the method in one embodiment of the invention;

Figure 11 illustrates a prior art combined vital signs display;

Figure 12 is a flow diagram of the method steps for alert generation based on the population model in one embodiment of the invention; and

Figure 13 is a flow diagram of the method steps for alert generation based on the patient-specific model in one embodiment of the invention.

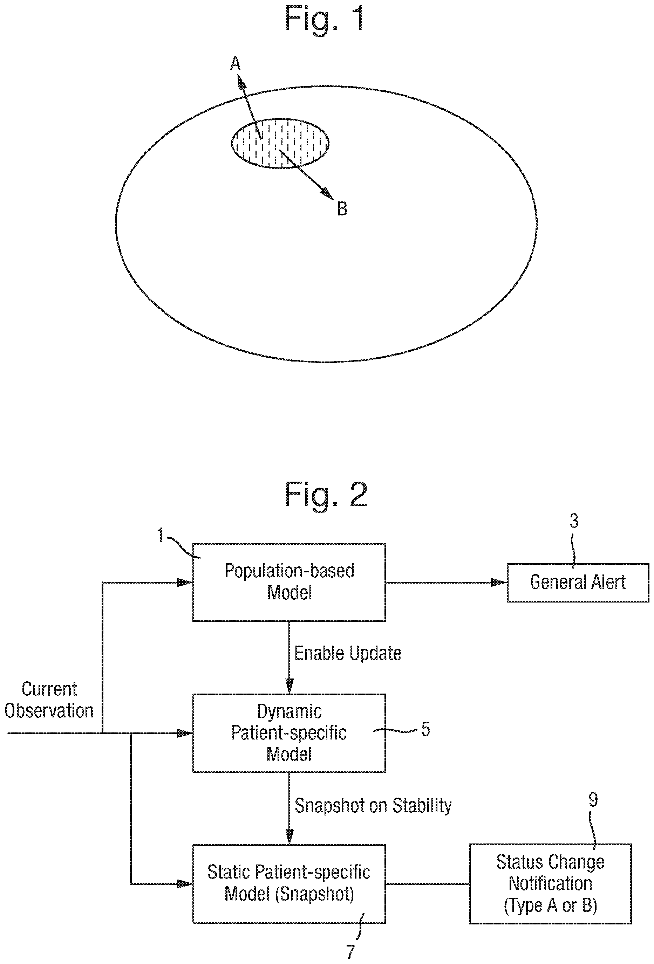

Figure 1 illustrates the concept of the operation of the monitoring system of the present invention once a patient-specific model has been learnt. The large ellipse represents schematically a multi-dimensional population model (for example the FDA-cleared Visensia model), represented as a two-dimensional schematic (in practice there are as many dimensions as measured parameters). By comparing a current observation to the population model as described in US-B2-7031857 it can be judged whether the current patient state is inside or outside the boundary of the ellipse which represents normality. If the patient's status is outside then the model threshold is exceeded and if this persists for a certain number of observations or a certain period, an alert is generated calling for intervention by a member of a medical emergency team.

The small ellipse within the larger ellipse in Figure 1 represents a patient-specific model that has been learnt during an initial phase of operation of the system. As will be explained in more detail below, on initiation of the system the patient-specific model has the same statistical properties as the global population-based model, but as data is acquired in real time, the updating of the patient's specific model causes its region of normality to shrink in size and define a region of normality specific to that patient. The smaller ellipse in Figure 1 represents that region of normality for the specific patient. After a fixed period of learning, for example 20 minutes, or after the region of normality defined by the patient-specific index has become stable, the patient-specific model can be frozen and then status change notifications generated by comparison of new vital signs measurements with the patient-specific model.

As illustrated in Figure 2, therefore, in this embodiment of the invention, a current observation (preferably having been pre-processed so that the parameters are normalised and decorrelated, e.g. by using a standard whitening process) is compared to the population-based model 1 and a general alert 3 can be generated if the predefined normality threshold (represented by the large ellipse in Figure 1) is exceeded. Thus a general alert derived from the population-based model can be generated at any time - even immediately on starting monitoring. Once the patient-specific model has been learnt in the initial phase, a static snapshot is taken of the patient-specific model, and incoming observations (again preferably pre-processed as mentioned above) are compared to the static snapshot of the patient-specific model in order to judge whether the observation represents normality for the specific patient (represented by the small ellipse in Figure 1) or a change of status. In the event of a status change, i.e. the reading lying outside a region of normality for that patient, a status change notification 9 can be generated, which may be according to Type A or Type B events as illustrated in Figure 1. The current observation can also be used to update the dynamic patient-specific model 5, assuming that updating is enabled (such enablement can be with reference to the population-based model as discussed in more detail below).

## Examples of updating patient-specific model

In this embodiment the population model (large ellipse in Figure 1) is estimated by a kernel density estimator from a large and artefact-free data set (as previously disclosed in European Patent EP 1 389 948 B1). The patient-specific model can also be estimated by a kernel density estimator and is updated by machine-learning techniques in an online fashion as explained below. Thus the population-based and patient-specific statistical models are probability density functions which give a probability density $p$ for each combination of measured vital signs $y$ (HR, BP, BR, $SpO_2$, Temp). The probability density functions in this embodiment are the sum of a plurality of Gaussian functions known as kernels, in a multi-dimensional parameter space (i.e. each point in that space represents a particular set of normalised vital signs measurements).

In one embodiment of the invention, the approach for updating the patient-specific model of vital signs is to employ particle filtering (sequential Monte Carlo technique), though by imposing some assumptions other machine-learning techniques such as Kalman Filtering or Unscented Kalman Filtering can be used (see: Beyond the Kalman Filter: Particle Filters for Tracking Applications , Branko Ristic, Artech House Publishers, 2004, ISBN 158053631X, or The unscented Kalman filter for nonlinear estimation, Wan E.A, and Van der Merwe, R, Adaptive Systems for Signal Processing, Communications, and Control Symposium 2000. AS-SPCC. The IEEE 2000).

A particle filter is a non-Gaussian density estimator, which is formulated in the Bayesian framework. Bayesian learning estimates the distribution of interest (posterior distribution), using the prior distribution and the likelihood of the current observation (more details on dynamic Bayesian updating can be found in, for example, Arulampalam, M.S.; Maskell, S.; Gordon, N.; Clapp, T., "A tutorial on particle filters for online nonlinear/non-Gaussian Bayesian tracking, "IEEE Transactions on Signal Processing, vol.50, no. 2, pp.174, 188, Feb 2002).

As mentioned above, the population distribution is used as the prior distribution for estimation of the posterior distribution for a specific patient. This allows measurement artefacts to be handled better and reduces the updating rate when the vital signs are well-away from the normal values. It also assists a better decision about the improvement or deterioration of the patient, for example, when the vital signs are moving away from the centre of normality of the patient model, but they are moving towards the centre of the normality of the population model as illustrated by arrow B in Figure 1.

Thus starting from the population distribution, to obtain the patient-specific distribution, suppose that the population distribution, i.e., the prior, is represented using a kernel density estimator such as Parzen window [see, e.g., US-B2-7031857]. Let $N_{\mathcal{P}}$ be the number of kernels, e.g. 400 in this embodiment, each of which is a Gaussian function

$$\mathcal{G}\left(x;\ \mu^j, C_{\mathcal{P}}\right) = \frac{1}{\sqrt{(2\,\pi)^d |C_{\mathcal{P}}|}} e^{-\frac{1}{2}\left(x-\mu^j\right)^T C_{\mathcal{P}}^{-1}\left(x-\mu^j\right)}$$

with mean $\mu^j$, $j = 1,..., N_{\mathcal{P}}$ and covariance $C_{\mathcal{P}}$, respectively. Here $d$ is the dimensionality of data. It is also possible to have different covariance matrices for each kernel, as in the Gaussian mixture models (GMM), but here for the sake of simplicity of notation, we assume that the covariance matrices are the same for all kernels. The population distribution that the process starts from is therefore given by:

$$p(x_t; \mathcal{P}) = \frac{1}{N_{\mathcal{P}}} \sum_{j=1}^{N_{\mathcal{P}}} \mathcal{G}\left(x_t; \mu_{\mathcal{P}}^j, C_{\mathcal{P}}\right) \qquad (1)$$

So the probability density $p$ for a particular set of values $x_t$ is the sum of the value of each of the Gaussian kernel. Furthermore, suppose that the vital signs and their noisy observations at time $t$ are denoted by $x_t$ and $y_t$, respectively, we have:

$$y_t = x_t + v_t \qquad (2)$$

where $v_t$ is the additive and zero-mean noise. Based on the Bayesian framework, we are interested in the estimation of the posterior distribution of the vital signs denoted by $p(x_t|y_t; \mathfrak{P})$ i.e. the probability of state $x_t$ given measurement $y_t$ for patient $\mathfrak{P}$. Suppose also that $x_t$ and $y_t$ obey the following Markovian state space:

$$x_t \sim p(x_t|x_{t-1}; \mathfrak{P}) \qquad (3)$$

$$y_t \sim p(y_t|x_t; \mathfrak{P}) \qquad (4)$$

i.e. that the values of $x_t$ are distributed according to $p(x_t|x_{t-1}; \mathfrak{P})$ and the observations $y_t$ are distributed according to $p(x_t|y_t; \mathfrak{P})$. The posterior distribution, i.e. the patient-specific distribution, is assumed to be non-Gaussian, and thus $N_{\mathfrak{P}}$ (e.g. 1000 in this embodiment) Gaussian kernels, each with potentially a different weight $w_t^i$ are used to approximate this distribution:

$$p(x_t|\ y_t; \mathfrak{P}) = \sum_{i=1}^{N_{\mathfrak{P}}} w_t^i\ \mathcal{G}\left(x_t; x_t^i, C_{\mathfrak{P}}\right) \qquad (5)$$

where $w_t^i$, $i = 1,..., N_{\mathfrak{P}}$ are kernel weights and $\mathcal{G}\left(x_t; x_t^i, C_{\mathfrak{P}}\right)$ is the Gaussian function centered at $x_t^i$ with covariance matrix $C_{\mathfrak{P}}$. Thus, instead of the Dirac delta function as in standard particle filter which is known as selective importance resampling (SIR), we use Gaussian kernels for approximation of posterior distribution, and therefore the regularised particle filter is employed. The regularised particle filter is the same as the SIR filter except it has a modified resampling technique to take into account the kernel used in the approximation of the posterior density [for more details see: Arulampalam, M.S.; Maskell, S.; Gordon, N.; Clapp, T., "A tutorial on particle filters for online nonlinear/non-Gaussian Bayesian tracking, "IEEE Transactions on Signal Processing, vol.50, no. 2, pp.174,188, Feb 2002].
Based on the above definitions and assumptions, an algorithm using a generic particle filter may be given by Algorithm 1.

Algorithm 1

```
FOR  i = 1: N_𝔅:
        -- Sample  x_0^i  from equation (1) and set the weight of each  w_0^i = 1/N_𝔅
```

ENDFOR [this gives us an initial model of posterior distribution consisting of, e.g. 1000, Gaussian kernels each centered at a particular value of $x_0^i$ and each of equal weight]

FOR $t = 1: T$ [at each receipt of a new observation]

```
        FOR  i = 1: N_𝔅
```

        -- sample from importance density $x_t^i \sim \pi(x_t | x_{t-1}^i, y_k)$

        -- calculate a new weight for each of the particle:

$$w_t^i \propto w_{t-1}^i \frac{p(y_t|x_t^i)p(x_t^i|x_{t-1}^i)}{\pi(x_t^i|x_{t-1},y_t)}$$ [see discussion below for how to calculate these terms]

```
        ENDFOR
```

-- normalise the weights $w_t^i = \frac{w_t^i}{\sum_{i=1}^{N_𝔅} w_t^i}$ to have unit sum

-- calculate $N_{eff} = \frac{1}{\sum_{i=1}^{N_𝔅}(w_t^i)^2}$ number of effective samples as a measure

for snapshots [It can happen that some of the weights are so low that the number of kernels with a sufficient weight to contribute to the model is too low. This resampling has the effect of introducing more relevant kernels into the patient-specific model and removing outlying kernels which have a very low weight.]

```
        IF  N_eff < N_T
```

        -- resample $\{x_t^i, w_t^i\}$ such that $w_t^i = \frac{1}{N_𝔅}$

```
        ENDIF
```

```
ENDFOR
```

In this algorithm, $\pi(x_t | n_{t-1}^i, y_k)$ is the importance density and the measurement transition probability is also given by:

$$p(y_t|x_t^i; \mathfrak{P}) \approx \mathcal{G}(y_t|0, C_v) \qquad (6)$$

where $\mathcal{G}(y_t|0,C_v)$ is the distribution of the additive noise $v_t$, which has been assumed to be distributed independently and identically. In this embodiment, the importance density is chosen to be equal to the prior density $\pi(x_t^i|x_{t-1}^i, y_t) \approx p(x_t^i|x_{t-1}^i)$, and therefore the update procedure for weights is simplified to (as in SIR filter):

$$w_t^i \propto w_{t-1}^i p(y_t|x_t^i) \qquad (7)$$

Optionally, the state transition probabilities can be approximated by the population model:

$$p\left(x_t^i \big| x_{t-1}^i; \mathfrak{P}\right) \approx p\left(x_t \big| x_{t-1}^i; \mathcal{P}\right) \qquad (8)$$

Here, we assume that the transition density is the same as the population density model given in equation (1). We may further approximate the state transition probability by:

$$p\left(x_t \big| x_{t-1}^i; \mathcal{P}\right) \approx \mathcal{G}\left(x_t; \mu_{\mathcal{P}}^{i,nst}, C_{\mathcal{P}}\right) \qquad (6)$$

where $\mu_{\mathcal{P}}^{i,nst} = \mathrm{argmax}_{\mu^j} \ \mathcal{G}\left(x_{t-1}^i; \mu^j, C_{\mathcal{P}}\right)$, for $j = 1, ..., N_{\mathcal{P}}$. That is to say, to sample $x_t^i$ from the population model, we sample from the kernel in the population model that is nearest to the particle $x_{t-1}^i$, or we consider the nearest kernel as the transition distribution. Equations (8) and (9) have the desirable effect that the patient-specific model's region of normality is always bounded by the population-based model. If a clinician decides, for a particular patient, that their region of normality lies outside the population-based region of normality, then this further approximation can be omitted.

Note that the measurement equation (2) is linear, but the transition of the state is non-Gaussian (nonlinear), since it is based on the population distribution, which is a non-Gaussian density. However, for a faster implementation, one may approximate the state transition by a Gaussian distribution and effectively employ the Kalman filter, which assumes Gaussian distribution for both initial and transition distributions.

Based on the above assumptions the implementation the generic algorithm given in Algorithm 1 is converted to Algorithm 2.

Algorithm 2

```
0) To get the initial patient-specific model randomly sample the
   population model to get N_𝔓 particles (e.g. N_𝔓 = 1000) each having an
```

associated weight $w_0^i = \frac{1}{N_{\mathfrak{P}}}$ centred at $x_0^i$ in parameter space, a "model" as defined by equation (5).

1) At time point $t$ receive an observation $y_t$

2) Construct the likelihood according to the equation (6) and update the weights in the patient specific model according to (7) (note the particle position in parameter space $x_0^i$ does not change, just the weight $w_t^i$.

3) Normalise the weights $w_t^i = \frac{w_t^i}{\sum_{i=1}^{N_{\mathfrak{P}}} w_t^i}$

4) Calculate number of inefficient samples and if they are less than a predefined threshold go to next step, otherwise go to step 1.

5) If it is desired for the patient-specific model to remain bounded by the population-based model, then resample from the population model (equation 8) i.e. for each particle find the nearest kernel in the population model and take a random sample from that kernel to be the new particle (equation 9). This changes the position of the particles (centre of the kernels), while the weights remain the same. This resampling also is independent from the observations.

6) Otherwise (ie if the patient-specific model may go outside the bounds of the population-based model) then use a standard resampling technique to have an un-weighted approximate density by eliminating particles having low importance weights and by replicating particles having high importance weights. This resampling makes all particles have the same weights.

7) The particles with new positions and new equal weights approximate the snapshot patient specific model.

As in the standard particle filter, the algorithm has some free parameters including the covariance of the process noise, the covariance of the measurement noise (if we assume they are Gaussian) and the number of effective samples. These parameters can be set empirically, according to the available data set in hand. A simple approach is to set the process noise covariance equal to the covariance of the kernel in the patient-specific model, and to set the measurement noise covariance equal to the covariance of the kernel of the population model.

**First numerical index**

A numerical index such as novelty score, e.g. the Visensia index (*VSI*) used by the applicant, is a measure for the wellness of the patient, and in US-B2-7031857 it was defined as a VSI score using equation (1) by:

$$VSI_{\mathcal{P}}(x_t) = -\log p(x_t; \mathcal{P}) + \log p(\mu_{t,\mathcal{P}}; \mathcal{P}) \qquad (7)$$

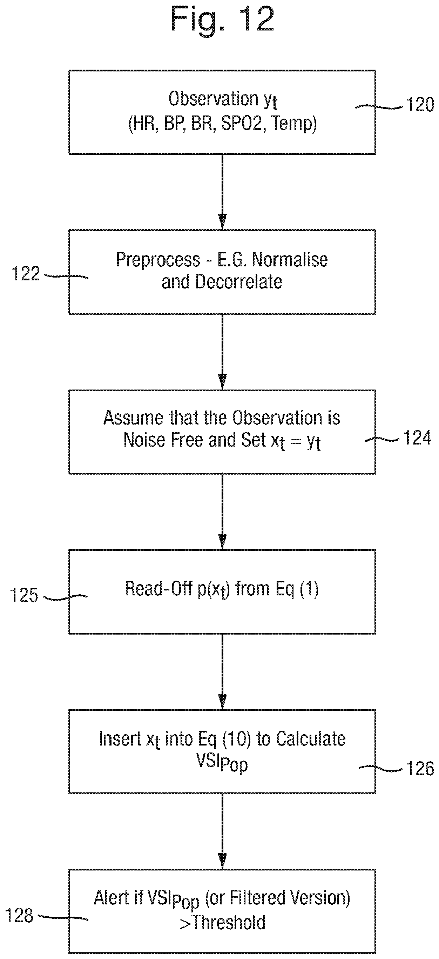

where $\mu_{t,\mathcal{P}}$ is the mean of $p(x_t; \mathcal{P})$. The term $\log p(\mu_{t,\mathcal{P}}; \mathcal{P})$ is added to make $VSI_{\mathcal{P}}(x_t)$ zero at the centre of the distribution. This same index is used in this embodiment as the first numerical index which is based on the population-based model. A threshold on this index defines the boundaries of the region regarded as normal and illustrated as the large ellipse in Figure 1. The population model is fixed so the mean, the second term in equation (10) does not change and can be pre-calculated from the model. As illustrated in Figure 12 obtaining the first numerical index simply involves taking the incoming multi-parameter observation $y_t$ (HR, BP, BR, SpO$_2$, Temp) at step 120, preprocessing it as mentioned above at step 122, then for this purpose the state $x_t$ is considered to be the same as the observation $y_t$ as indicated at step 124. This is then compared in step 125 to the population model of equation (1) to read off its probability density $p(x_t|y_t)$ and this is inserted this into equation (10) to calculate $VSI_{\mathcal{P}}$ in step 126. In step 128 an alert can be generated if $VSI_{\mathcal{F}}$ or a filtered version of it is outside a threshold, i.e. outside the large ellipse of Figure 1.

**Second numerical index**

To obtain a second numerical index, based on the patient specific model, two approaches, as explained below, can be used to calculate the novelty score and therefore generate relevant alerts.

**Approach 1**

Similar to equation (10), a novelty (VSI) score for the patient-specific model can be defined (which sets the boundaries of the small ellipse in Figure 1):

$$VSI_{\mathfrak{P}}(x_t) = -\log p(x_t|y_t; \mathfrak{P}) + \log p(\mu_{t,\mathfrak{P}}; \mathfrak{P}) \qquad (8)$$

where $\mu_{t,\mathfrak{P}}$ is the mean of $p(x_t|y_t; \mathfrak{P})$. This definition sets the boundaries of the small ellipse in Figure 1.

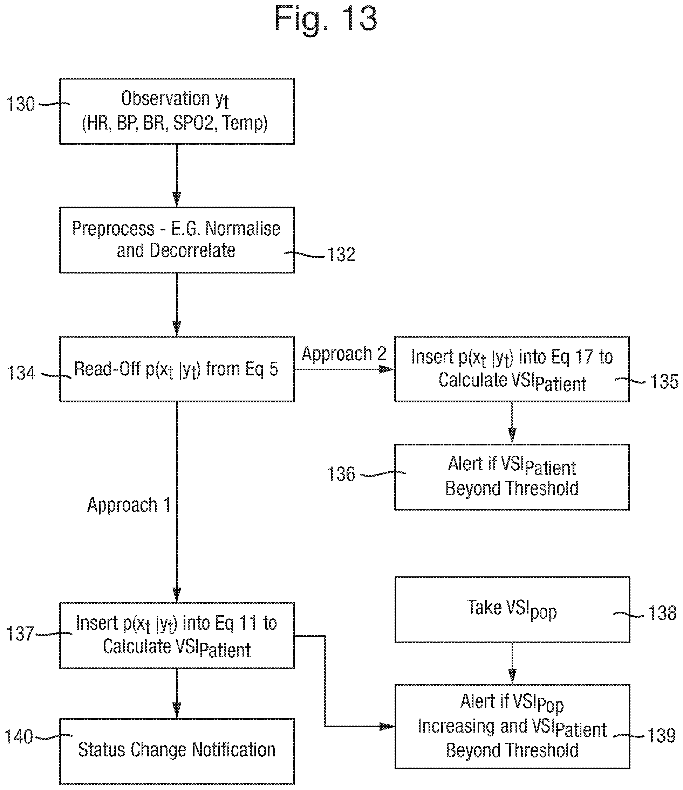

Thus to obtain the second numerical index, as illustrated in Figure 13, the incoming multi-parameter observation $y_t$ (HR, BP, BR, SpO$_2$, Temp) received at step 130, is pre-processed in step 132, and this is compared in step 134 to the current snapshot of the patient-specific model of equation (5) to obtain its probability density $p(x_t|y_t; \mathfrak{P})$ inserting this into equation (11) in step 136. Again the second term based on the mean of the model is fixed (once the patient-specific model is frozen - its mean would be recalculated when it is updated) and can be pre-calculated.

If the value of personalised $VSI_{\mathfrak{P}}$ exceeds a threshold this is used to operate a status change notification in step 140 which can be displayed or transmitted to a clinician, who then determines whether the patient has deteriorated or improved. In the case of deterioration, as shown in arrow A in Figure 1, this would provide an earlier warning than the standard population model as it will be clear from Figure 1 that a change as shown by arrow A will operate a status change notification before an alarm is operated on crossing the boundary of the large ellipse. In the case of improvement, as shown by arrow B in Figure 1, the system can permit a new stage of machine learning, to track the new and improved state.

- With an A type notification, patient deterioration is indicated, as the global VSI score will have increased with respect to the mean VSI score for the learned model. This type of notification will likely occur earlier than an eventual Visensia alert, because the state has not yet exceeded first (population) threshold - the outer ellipse in Figure 1;
- With a B type notification, the global VSI score will have decreased - indicative of an improvement of the patient's condition.
- Steps 138 and 139 illustrate automatic use of the global $VSI_{\mathcal{F}}$ to determine whether an alert should be generated based on the learned (patient-specific) $VSI_{\mathfrak{P}}$ score going beyond its threshold.

It is envisaged that either type of notification would summon a clinician to make an assessment of what has changed. In the event of a type A alert, this could warrant escalation of the intervention, and will have therefore provided an earlier warning than the original system. In the event of a B type alert, the option exists for the clinician to authorise the system to re-engage the learning process, in order to track the patient's improvement.

In another scenario the patient state might change along a trajectory that leads to only small changes (+ or -) in VSI. In this case, the system can also be allowed to learn the patient's new state.

**Approach 2**

As a more sophisticated alternative, the *VSI* for personalised monitoring can consider both population and patient models, which leads to the second approach. By assuming that these distributions are independent, we may write:

$$p(x_t; \mathfrak{P}, \mathcal{P}) \propto p(x_t | y_t; \mathfrak{P}) p(x_t; \mathcal{P}) \qquad (9)$$

This leads to the definition of the personalised *VSI* for a specific patient $\mathfrak{P}$:

$$VSI_{\mathfrak{P}}(x_t) \propto -\log p(x_t; \mathfrak{P}, \mathcal{P}) + \log p(\mu_{t,\mathfrak{P}\mathcal{P}}; \mathfrak{P}, \mathcal{P}) \qquad (10)$$

where $\mu_{t,\mathfrak{P}\mathcal{P}}$ is the mean of $p(x_t; \mathfrak{P}, \mathcal{P})$. Same as above, the term $\log p(\mathfrak{P}, \mathcal{P})$ has been added to make $VSI_{\mathfrak{P}(x_t)}$ zero at the centre (mean) of the distribution. It is clear that if the values of both population and patient probability density values decrease, which is a sign of deterioration, the *VSI* increases, whereas if the probability density values increase, which is a sign of improvement, the *VSI* decreases.

Based on equation (12), and according to the equations (1) and (5), after resampling we have:

$$p(x_t; \mathfrak{P}, \mathcal{P}) \propto \frac{1}{N_{\mathfrak{P}}} \sum_{i=1}^{N_{\mathfrak{P}}} \mathcal{G}(x_t; x_t^i, C_{\mathfrak{P}}) \frac{1}{N_{\mathcal{P}}} \sum_{j=1}^{N_{\mathcal{P}}} \mathcal{G}(x_t; \mu^j, C_{\mathcal{P}}) \qquad (11)$$

Since the product of two Gaussian probability density functions (pdf) is Gaussian itself, this pdf is a Gaussian mixture model. By algebraic manipulation of equation (14), we obtain:

$$p(x_t; \mathfrak{P}, \mathcal{P}) \propto \sum_{i=1}^{N_{\mathfrak{P}}} \sum_{j=1}^{N_{\mathcal{P}}} \mathcal{G}(x_t; \mu_{t,\mathfrak{P}\mathcal{P}}^{i,j}, C_{\mathfrak{P}\mathcal{P}}) \qquad (12)$$

where

$$\mu_{t,\mathfrak{P}\mathcal{P}}^{i,j} = \left(C_{\mathfrak{P}}^{-1} + C_{\mathcal{P}}^{-1}\right)^{-1} \left(C_{\mathfrak{P}\mathcal{P}}^{-1} x_t^i + C_{\mathfrak{P}\mathcal{P}}^{-1} \mu^j\right), \quad C_{\mathfrak{P}\mathcal{P}} = \left(C_{\mathfrak{P}}^{-1} + C_{\mathcal{P}}^{-1}\right)^{-1} \qquad (13)$$

If it is assumed that the covariance is spherical, the above mean basically is the average of the means of population and patient pdf weighted according to their variance. Therefore, the personalised Visensia index is defined by:

$$VSI_{\mathfrak{P}}(x_t) = -\log p(x_t | y_t; \mathfrak{P}) p(x_t; \mathcal{P}) + \log p\left(\frac{1}{N_{\mathcal{P}}N_{\mathfrak{P}}} \sum_{i=1}^{N_{\mathcal{P}}} \sum_{j=1}^{N_{\mathfrak{P}}} \mu_{t,\mathfrak{P}\mathcal{P}}^{i,j}; \mathfrak{P}, \mathcal{P}\right) \qquad (14)$$

which can be used as a measure for the wellness of the patient.

Thus to obtain the second numerical index the incoming multi-parameter observation $y_t$ (HR, BP, BR, SpO$_2$, Temp), is compared to the population model of equation (1) to read off its probability density $p(x_t; \mathcal{P})$ with respect to the population and to the patient-specific model of equation (5) to obtain its probability density $p(x_t|y_t; \mathfrak{P})$ with respect to the patient's own status inserting this into equation (17) This is illustrated in step 135 in Figure 13. Again the second term based on the mean of the two models is fixed (once the patient-specific model is frozen - its mean would be recalculated when it is updated) and can be pre-calculated. If the combined index (or a smoothed version of it) exceeds a specified threshold, then an alert is generated at step 136.

**Artefact Removal**

Artefacts such as motion of probe detachment, or movement can provide spurious measurements and would result, generally, in a high instantaneous VSI value in the population model (which would normally be smoothed out to prevent false alerts). To stop updating the model based on the noisy measurements, the artefact-free data may be selected by clinicians or automatically. Automatic selection of the acquired data would be on the basis of the population model. For instance, acquired vital sign sets can be excluded by the following rules:

1. Initially anything that would generate an alert based on the population model is excluded from the online learning.

2. After a short time, sequences of VSI values have been acquired with a distribution about some mean value. At this point, further data points can be excluded based on this distribution.

3. If exclusion on the above basis persists, this may be indicative of the fact that the patient's status is changing, and the learning would be re-initialised to step 1.

It may be the case that a patient's state is stable, but the vital signs are so abnormal that they would always generate a VSI alert, for example, if the heart rate is over 150, but the patient is deemed by a clinician to be "stable". With the learning system proposed above, the specific model would never learn. However, the acceptance for online learning criterion can be modified by allowing the operator (e.g. clinician) to treat certain vital signs (in this case the Heart Rate) to be treated as "missing variables" for the purpose of accepting the measurements. A simple method for doing this is to replace that variable's value by the population mean value.

Therefore, in this approach when the vital signs are considerably small or large, the system stops updating the weights of kernels, as these parameters could be because of the artefact in the data set. This is not however a problem, in the updating example based on the particle filtering, the particles are sampled from the prior distribution.

## Examples

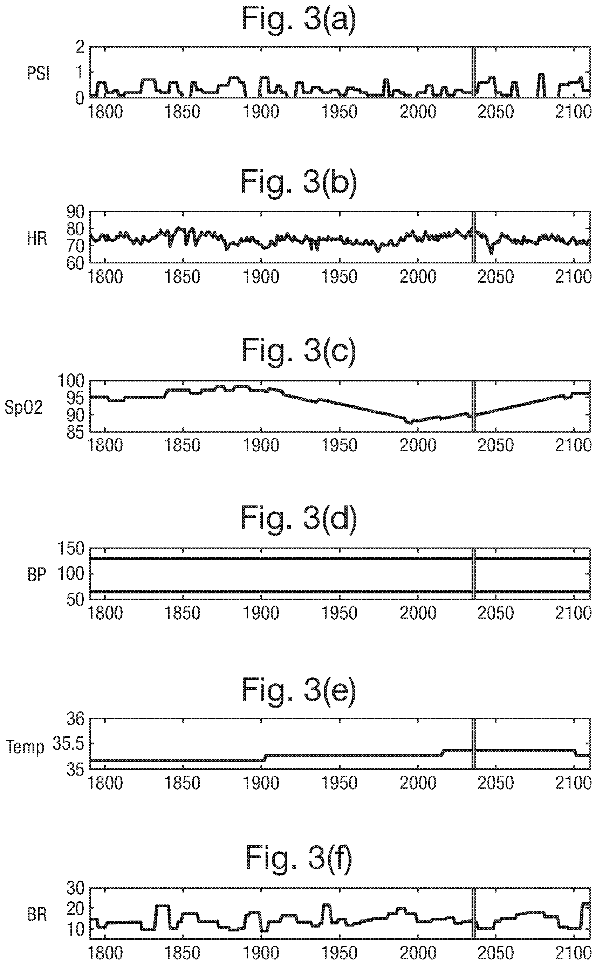

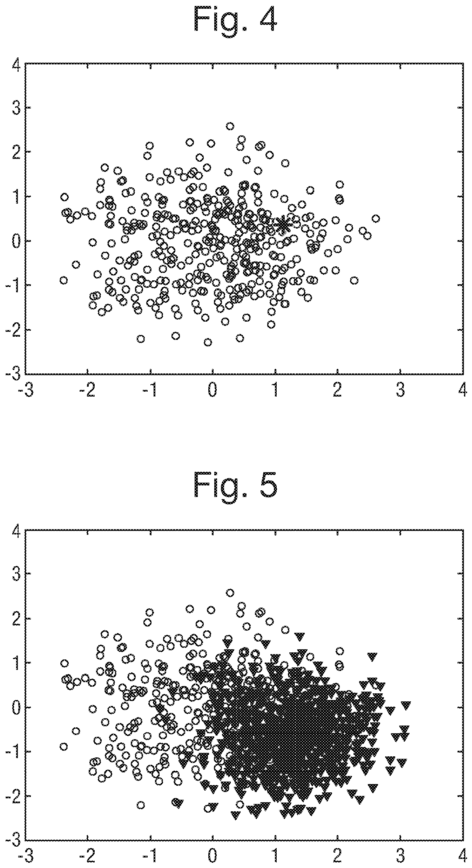

In this section, we provide some of examples of applying the invention to real vital signs measurements. Figures 3(a) to (f) are examples of the population-based index (a) and of five vital signs; heart rate (HR), oxygen saturation (SpO2), blood pressure (BP), temperature (Temp) and breathing rate (BR) (b)-(f) The "current observation" has been shown by a solid vertical line. Figure 4 shows the first two principal components of the population-based statistical model, where the centre of the kernels is shown by circles, and the principal components of the current observation are shown by the large asterisk. In this example, the observations were first normalised (or they can be whitened) and we set the number of particles equal to 1000, the observation and transition noises were assumed to be white Gaussian with powers equal to 0.1 and 1, respectively.

The first two principal components of the centre of the kernels modelled by particles are shown again in Figure 5 for population-based statistical model (circles), together with the centre of the Gaussian kernels (particles) for the patient-specific statistical model using filled triangles. The mean or mode of kernels represented by triangles can be considered as the normal value of vital signs for that specific patent at a given time.

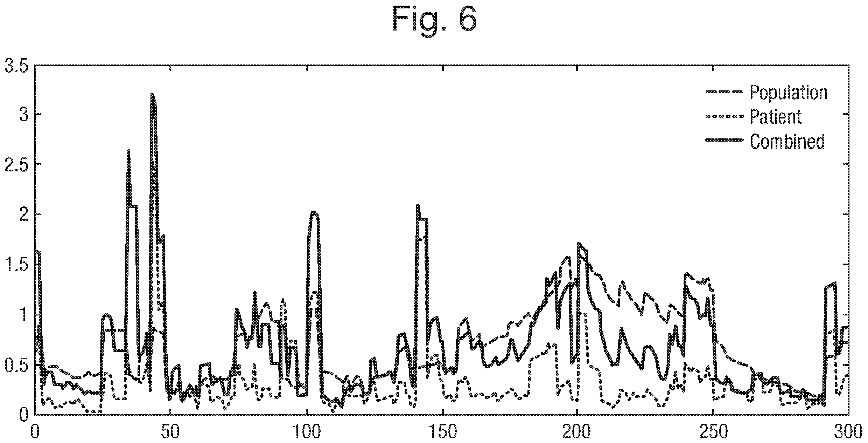

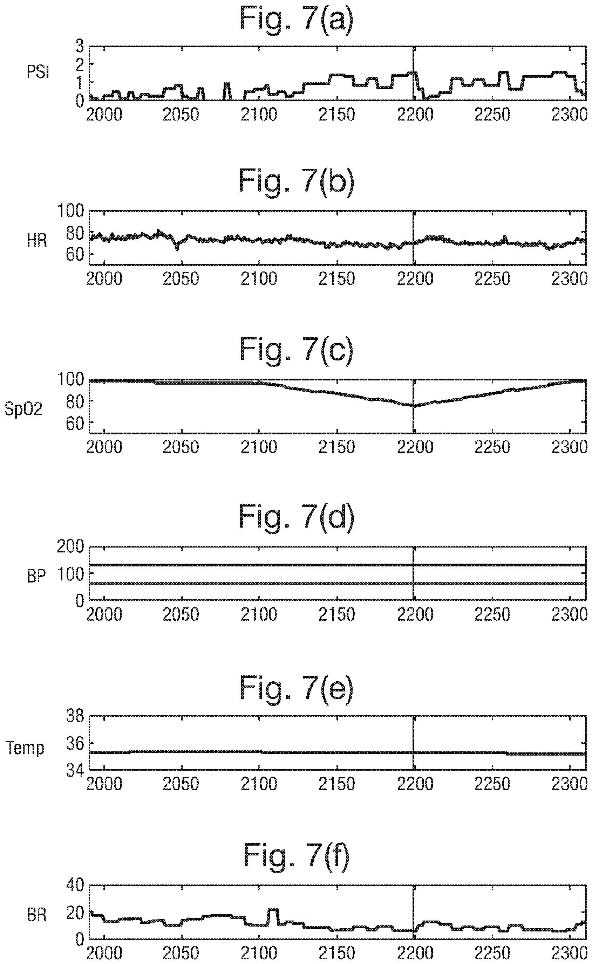

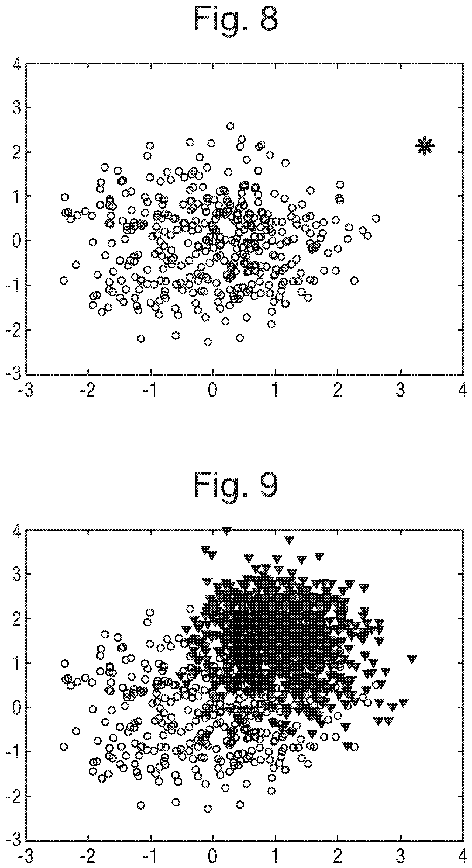

The novelty scores calculated with respect to the population -based statistical model (equation 11) patient (equation 13) and combined (equation 17) are shown in Figure 6. If the patient and population novelty scores increase, the combined novelty score increases as well. The combined novelty score can be less that the patient-specific model based novelty score, if the vital signs move enough towards the centre of the population-based model. Figure 7(a) to (f) shows an example of the population-based index and vital signs when the current observation is well-outside of the boundaries of the population-based statistical model (star in the top right corner in Figure 8). In this case the patient-specific model (circles) still is inside of the population model (triangles), see Figure 9. As explained above, this is because of the proposed state transition which lowers the impact of the outliers on the learning procedure. Note that there are some outliers outside of the population model, which are the result of sampling procedure which adds some noise to the new samples.

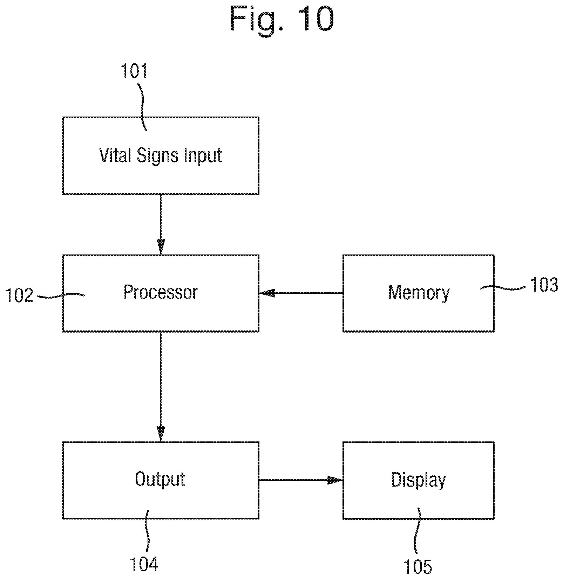

The invention may be embodied in a dedicated vital signs monitor or may be embodied in a computer system. In both cases the main parts of the system are, as illustrated in Figure 10, an input 101 for receiving the five vital signs, a processor 102 for processing them and comparing them to the statistical models stored in memory 103 to produce numerical indices, and an output 104 which may include a display 105 for displaying the numerical indices, normally together with the individual vital signs themselves. The processor 102 is also responsible for establishing and then updating the patient-specific model in line with the update procedure described above.

## Claims

1. A method of monitoring the status of a patient comprising the steps of:

continuously measuring a plurality of patient vital signs to provide a succession of multi-parameter vital signs observations (130),
comparing each successive multi-parameter vital signs observation (130) to a first statistical model (1) being a population-based reference model (1) mapping the probability distribution of multi-parameter vital signs observations for a population of different individuals,
calculating a first numerical index based on the probability density of the current multi-parameter vital signs observation (130) obtained by comparison with the first statistical model (1), wherein an increased value of said index represents an increase in abnormality with respect to the first statistical model (1), and

outputting, on an apparatus output, an alert notification if the first numerical index exceeds a first threshold; **characterized by**:

comparing each successive multi-parameter vital signs observation (130) to a second statistical model (5, 7) being a patient-specific model which is a probability distribution based at least partly on previous multi-parameter vital signs observations for the patient being monitored,

calculating a second numerical index based on the probability density of the current multi-parameter vital signs observation (130) obtained by comparison with the second statistical model (5, 7), wherein an increased value of said index represents an increase in abnormality with respect to the statistical model (5, 7), and

outputting a status change notification if the second numerical index exceeds a second threshold;

and by the method further comprising the step of updating the patient-specific model (5) using the most recent multi-parameter vital signs observation (130) only when the comparison of the most recently received multi-parameter vital signs observation (130) with the population-based model (1) indicates that the observation is within a predefined normal region of the population-based model (1).

2. A method according to claim 1 wherein step of updating the patient-specific model (5, 7) is stopped when the patient-specific model (5, 7) is stable, and restarted when comparison of the most recent multi-parameter vital signs observation (130) with the patient-specific model (5, 7) indicates a change in patient status.

3. A method according to claim 1 or 2 wherein the status change notification is only generated if said change corresponds to an increase in the first numerical index, generated with reference to the reference model (1), thereby indicative of a deterioration in the patient's condition.

4. A method according to claim 3 wherein the increase of the first numerical index is judged by comparing the value of the first numerical index obtained for the current multi-parameter vital signs observation (130) to the value of the first numerical index obtained when the patient-specific model (5, 7) became stable.

5. A method according to any one of the preceding claims, wherein the patient specific model (5, 7) is updated by a Bayesian process comprising calculating from the patient-specific model (5, 7) the likelihood of the most recent multi-parameter vital signs observation, multiplying the model's probability distribution by the calculated likelihood and renormalizing it to form an updated probability distribution for the model (5, 7).

6. A method according to claim 5 wherein the patient-specific model (5, 7) consists of a plurality of particles, being samples initially generated from the population-based model (1) by random sampling, and the Bayesian update process comprises adding a noise term to each of the particles, computing each particle's likelihood on receipt of a multi-parameter vital signs observation, and then resampling the plurality of particles wherein the probability of selection of the particle is proportional to its computed likelihood.

7. A method according to any one of the preceding claims wherein updating of the patient-specific model (5, 7) is selectively allowable by a clinician even if the population-based model (1) is showing abnormality.

8. A method according to claim 6 wherein resampling of the plurality of the particles is performed by sampling the nearest kernel in the population-based model (1) to each particle wherein the probability of selection of the particle is proportional to its computed likelihood, thus ensuring that the patient specific model (5, 7) remains bounded by the population based model (1).

9. A method according to 7 wherein the clinician can instruct the measurement of one or more vital signs to be ignored, these being treated as missing variables in determining whether to update the patient-specific model (5, 7).

10. A method according to any one of the preceding claims, wherein the predefined region of normality for determining whether to update the patient-specific model (5, 7) is different from the region defined by the threshold for determining whether to generate an alert notification.

11. A method according to any one of the preceding claims wherein an alert or status change notification is only generated if the respective threshold is exceeded for more than a predefined number of multi-parameter vital signs observations which are successive or within a predetermined time period.

12. A method according to claim 11, wherein the alert is triggered by thresholding a combined measure derived from both the population-based model (1) and the patient-specific model (5, 7).

13. A method according to any one of the preceding claims wherein the vital signs measured comprise heart rate, breathing rate, blood pressure, blood oxygen saturation and body temperature.

14. An apparatus for executing the method of any one of the preceding claims, the apparatus comprising an input (101) for receiving the patient's vital signs measurements, a memory (103) storing the statistical models, a processor (102) programmed to execute the comparing and calculating steps and an output to output the alert and status change notifications.

15. Apparatus according to claim 14 wherein the output comprises a display (105) for displaying the notifications, the display (105) being adapted to display the measured vital signs and at least one of the first and second numerical indices.

**Patentansprüche**

1. Verfahren des Überwachens des Zustands eines Patienten, umfassend die folgenden Schritte:

   Fortlaufendes Messen einer Vielzahl von Lebenszeichen des Patienten, um eine Abfolge von Mehrparameter-Lebenszeichen-Beobachtungen (130) bereitzustellen,
   Vergleichen von jeder aufeinanderfolgenden Mehrparameter-Lebenszeichen-Beobachtung (130) mit einem ersten statistischen Modell (1), das ein Populations-basiertes Referenzmodell (1) ist, das die Wahrscheinlichkeitsverteilung von Mehrparameter-Lebenszeichen-Beobachtungen für eine Population aus unterschiedlichen Individuen aufzeichnet,
   Berechnen eines ersten numerischen Indexes beruhend auf der Wahrscheinlichkeitsdichte der aktuellen Mehrparameter-Lebenszeichen-Beobachtung (130), die durch den Vergleich mit dem ersten statistischen Modell (1) erhalten wurde, wobei ein erhöhter Wert des Indexes eine Erhöhung der Anomalie mit Bezug auf das erste statistische Modell (1) darstellt, und
   Ausgeben, auf einem Vorrichtungsausgang, einer Warnmeldung, wenn der erste numerische Index einen ersten Schwellenwert übersteigt;
   **gekennzeichnet durch**:

   Vergleichen von jeder aufeinanderfolgenden Mehrparameter-Lebenszeichen-Beobachtung (130) mit einem zweiten statistischen Modell (5, 7), das ein patientenspezifisches Modell ist, das eine Wahrscheinlichkeitsverteilung ist, mindestens teilweise beruhend auf vorherigen Mehrparameter-Lebenszeichen-Beobachtungen für den überwachten Patienten,
   Berechnen eines zweiten numerischen Indexes beruhend auf der Wahrscheinlichkeitsdichte der aktuellen Mehrparameter-Lebenszeichen-Beobachtung (130), die durch den Vergleich mit dem zweiten statistischen Modell (5, 7) erhalten wurde, wobei ein erhöhter Wert des Indexes eine Erhöhung der Anomalie mit Bezug auf das statistische Modell (5, 7) darstellt, und
   Ausgeben einer Zustandsänderungsmeldung, wenn der zweite numerische Index einen zweiten Schwellenwert übersteigt;

   und **dadurch, dass** das Verfahren ferner den Schritt des Aktualisierens des patientenspezifischen Modells (5) unter Verwendung der aktuellsten Mehrparameter-Lebenszeichen-Beobachtung (130) nur dann umfasst, wenn der Vergleich der aktuellsten erhaltenen Mehrparameter-Lebenszeichen-Beobachtung (130) mit dem Populations-basierten Modell (1) anzeigt, dass die Beobachtung in einem festgelegten normalen Bereich des Populations-basierten Modells (1) liegt.

2. Verfahren nach Anspruch 1, wobei der Schritt des Aktualisierens des patientenspezifischen Modells (5, 7) angehalten wird, wenn das patientenspezifische Modell (5, 7) stabil ist, und neu gestartet wird, wenn der Vergleich der aktuellsten Mehrparameter-Lebenszeichen-Beobachtung (130) mit dem patientenspezifischen Modell (5, 7) eine Änderung des Patientenzustands anzeigt.

3. Verfahren nach Anspruch 1 oder 2, wobei die Zustandsänderungsmeldung nur dann erzeugt wird, wenn die Änderung einer Erhöhung in dem ersten numerischen Index entspricht, erzeugt mit Bezug auf das Referenzmodell (1), wodurch eine Verschlechterung des Zustands des Patienten angezeigt wird.

4. Verfahren nach Anspruch 3, wobei die Erhöhung des ersten numerischen Indexes durch das Vergleichen des Werts des ersten numerischen Indexes, der für die aktuelle Mehrparameter-Lebenszeichen-Beobachtung (130) erhalten wird, mit dem Wert des ersten numerischen Indexes beurteilt wird, der erhalten wird, wenn das patientenspezifische Modell (5, 7) stabil wird.

5. Verfahren nach einem der vorstehenden Ansprüche, wobei das patientenspezifische Modell (5, 7) durch ein Bayesisches Verfahren aktualisiert wird, umfassend das Berechnen von dem patientenspezifischen Modell (5, 7) der Wahrscheinlichkeit der aktuellsten Mehrparameter-Lebenszeichen-Beobachtung, das Multiplizieren der Wahrscheinlichkeitsverteilung des Modells mit der berechneten Wahrscheinlichkeit und das Renormalisieren, um eine aktualisierte Wahrscheinlichkeitsverteilung für das Modell (5, 7) zu bilden.

6. Verfahren nach Anspruch 5, wobei das patientenspezifische Modell (5, 7) aus einer Vielzahl von Teilchen besteht, die Proben sind, die anfänglich von dem Populations-basierten Modell (1) durch Stichproben erzeugt werden, und das Bayesische Aktualisierungsverfahren das Hinzufügen eines Geräuschbegriffs zu jedem der Teilchen, das Berechnen der Wahrscheinlichkeit jedes Teilchens des Erhalts einer Mehrparameter-Lebenszeichen-Beobachtung und die anschließende erneute Probe der Vielzahl von Teilchen umfasst, wobei die Wahrscheinlichkeit der Auswahl des Teilchens proportional zu der berechneten Wahrscheinlichkeit ist.

7. Verfahren nach einem der vorstehenden Ansprüche, wobei das Aktualisieren des patientenspezifischen Modells (5, 7) von einem Kliniker selektiv zulässig ist, selbst dann, wenn da Populations-basierte Modell (1) eine Anomalie zeigt.

8. Verfahren nach Anspruch 6, wobei die erneute Probe der Vielzahl der Teilchen durch die Probe des nächsten Kerns in dem Populations-basierten Modell (1) zu jedem Teilchen durchgeführt wird, wobei die Wahrscheinlichkeit der Auswahl proportional zu der berechneten Wahrscheinlichkeit ist, wodurch sichergestellt wird, dass das patientenspezifische Modell (5, 7) durch das Populations-basierte Modell (1) begrenzt bleibt.

9. Verfahren nach Anspruch 7, wobei der Kliniker anweisen kann, dass die Messung von einem oder mehreren Lebenszeichen ignoriert wird, wobei diese als fehlende Variablen beim Bestimmen, ob das patientenspezifische Modell (5, 7) aktualisiert werden soll, behandelt werden.

10. Verfahren nach einem der vorstehenden Ansprüche, wobei der vordefinierte Bereich der Normalität zum Bestimmen, ob das patientenspezifische Modell (5, 7) aktualisiert werden soll, sich von dem Bereich unterscheidet, der durch den Schwellenwert zum Bestimmen, ob eine Warnmeldung erzeugt werden soll, definiert wird.

11. Verfahren nach einem der vorstehenden Ansprüche, wobei eine Warn- oder Zustandsänderungsmeldung nur dann erzeugt wird, wenn der entsprechende Schwellenwert für mehr als eine festgelegte Anzahl von Mehrparameter-Lebenszeichen-Beobachtungen überschritten wird, die aufeinanderfolgend sind oder in einem festgelegten Zeitraum liegen.

12. Verfahren nach Anspruch 11, wobei die Warnung durch die Schwellenwertbildung eines kombinierten Werts ausgelöst wird, der von dem Populations-basierten Modell (1) sowie dem patientenspezifischen Modell (5, 7) stammt.

13. Verfahren nach einem der vorstehenden Ansprüche, wobei die gemessenen Lebenszeichen Herzfrequenz, Atemfrequenz, Blutdruck, Blutsauerstoffsättigung und Körpertemperatur umfassen.

14. Vorrichtung zum Ausführen des Verfahrens nach einem der vorstehenden Ansprüche, wobei die Vorrichtung eine Eingabe (101) zum Aufnehmen der Messungen der Lebenszeichen des Patienten, einen Speicher (103), der die statistischen Modelle speichert, einen Prozessor (102), der programmiert ist, die Vergleichs- und Berechnungsschritte durchzuführen, und eine Ausgabe zum Ausgeben der Warn- und Zustandsänderungsmeldung umfasst.

15. Vorrichtung nach Anspruch 14, wobei die Ausgabe eine Anzeige (105) zum Anzeigen der Meldungen umfasst, wobei die Anzeige (105) eingerichtet ist, die gemessenen Lebenszeichen und mindestens einen der ersten und zweiten numerischen Indizes anzuzeigen.

**Revendications**

1. Procédé de surveillance du statut d'un patient comprenant les étapes de :

mesure en continu d'une pluralité de signes vitaux d'un patient pour produire une succession d'observations de signes vitaux à paramètres multiples (130),

comparaison de chaque observation successive de signes vitaux à paramètres multiples (130) à un premier modèle statistique (1) étant un modèle de référence fonction de la population (1) dressant une carte de la répartition de probabilité d'observations de signes vitaux à paramètres multiples pour une population de différents individus,

calcul d'un premier indice numérique en fonction de la densité de probabilité de l'observation actuelle de signes vitaux à paramètres multiples (130) obtenue par comparaison avec le premier modèle statistique (1), une valeur augmentée dudit indice représentant une augmentation d'une anomalie par rapport au premier modèle statistique (1), et

émission, sur une sortie d'appareil, d'une notification d'alerte si le premier indice numérique dépasse un premier seuil ;

**caractérisé par** :

la comparaison de chaque observation successive de signes vitaux à paramètres multiples (130) à un deuxième modèle statistique (5, 7) étant un modèle spécifique au patient qui est une répartition de probabilité en fonction au moins en partie de précédentes observations de signes vitaux à paramètres multiples pour le patient sous surveillance,

le calcul d'un deuxième indice numérique en fonction de la densité de probabilité de l'observation actuelle de signes vitaux à paramètres multiples (130) obtenue par comparaison avec le deuxième modèle statistique (5, 7), une valeur augmentée dudit indice représentant une augmentation d'une anomalie par rapport au modèle statistique (5, 7), et

l'émission d'une notification de changement de statut si le deuxième indice numérique dépasse un deuxième seuil ;

et par le procédé comprenant en outre l'étape de mise à jour du modèle spécifique au patient (5) à l'aide de l'observation la plus récente de signes vitaux à paramètres multiples (130) seulement lorsque la comparaison de l'observation la plus récemment reçue de signes vitaux à paramètres multiples (130) avec le modèle fonction de la population (1) indique que l'observation se situe dans une région normale prédéfinie du modèle fonction de la population (1).

2. Procédé selon la revendication 1, dans lequel l'étape de mise à jour du modèle spécifique au patient (5, 7) est arrêtée lorsque le modèle spécifique au patient (5, 7) est stable, et redémarrée lorsque la comparaison de l'observation la plus récente de signes vitaux à paramètres multiples (130) avec le modèle spécifique au patient (5, 7) indique un changement du statut du patient.

3. Procédé selon la revendication 1 ou 2, dans lequel la notification de changement de statut est seulement générée si ledit changement correspond à une augmentation du premier indice numérique, généré par rapport au modèle de référence (1), révélant par conséquent une détérioration de l'état du patient.

4. Procédé selon la revendication 3, dans lequel l'augmentation du premier indice numérique est jugée en comparant la valeur du premier indice numérique obtenue pour l'observation actuelle de signes vitaux à paramètres multiples (130) avec la valeur du premier indice numérique obtenue lorsque le modèle spécifique au patient (5, 7) est devenu stable.

5. Procédé selon l'une quelconque des revendications précédentes, dans lequel le modèle spécifique au patient (5, 7) est mis à jour par un processus de Bayes comprenant le calcul à partir du modèle spécifique au patient (5, 7) de la probabilité de l'observation la plus récente de signes vitaux à paramètres multiples, la multiplication de la répartition de probabilité du modèle par la probabilité calculée et sa renormalisation pour former une répartition de probabilité mise à jour pour le modèle (5, 7).

6. Procédé selon la revendication 5, dans lequel le modèle spécifique au patient (5, 7) est composé d'une pluralité de particules, étant des échantillons initialement générés à partir du modèle fonction de la population (1) par un échantillonnage aléatoire, et le processus de mise à jour de Bayes comprend l'ajout d'un terme de bruit à chacune des particules, le calcul de la probabilité de chaque particule à la réception d'une observation de signes vitaux à paramètres multiples et puis le rééchantillonnage de la pluralité de particules, la probabilité de sélection de la particule étant proportionnelle à sa probabilité calculée.

7. Procédé selon l'une quelconque des revendications précédentes, dans lequel la mise à jour du modèle spécifique au patient (5, 7) peut être autorisée de manière sélective par un clinicien même si le modèle fonction de la population (1) indique une anomalie.

8. Procédé selon la revendication 6, dans lequel le rééchantillonnage de la pluralité de particules est effectué par l'échantillonnage du noyau le plus proche dans le modèle fonction de la population (1) de chaque particule, la probabilité de sélection de la particule étant proportionnelle à sa probabilité calculée, garantissant ainsi que le modèle spécifique au patient (5, 7) reste limité par le modèle fonction de la population (1).

9. Procédé selon la revendication 7, dans lequel le clinicien peut ordonner que la mesure d'au moins un signe vital soit ignorée, celui-ci étant traité comme variable manquante dans la détermination de savoir si l'on doit mettre à jour le modèle spécifique au patient (5, 7).

10. Procédé selon l'une quelconque des revendications précédentes, dans lequel la région prédéfinie de normalité destinée à déterminer si l'on doit mettre à jour le modèle spécifique au patient (5, 7) est différente de la région définie par le seuil destinée à déterminer si l'on doit générer une notification d'alerte.

11. Procédé selon l'une quelconque des revendications précédentes, dans lequel une notification d'alerte ou de changement de statut est seulement générés si le seuil respectif est dépassé de plus d'un nombre prédéfini d'observations de signes vitaux à paramètres multiples qui sont successives ou dans une période de temps prédéterminée.

12. Procédé selon la revendication 11, dans lequel la notification est déclenchée par seuillage d'une mesure combinée à partir à la fois du modèle fonction de la population (1) et du modèle spécifique au patient (5, 7).

13. Procédé selon l'une quelconque des revendications précédentes, dans lequel les signes vitaux mesurés comprennent le rythme cardiaque, le rythme respiratoire, la tension artérielle, la saturation en oxygène sanguin et la température corporelle.

14. Appareil destiné à effectuer le procédé selon l'une quelconque des revendications précédentes, l'appareil comprenant une entrée (101) destinée à recevoir les mesures de signes vitaux d'un patient, une mémoire (103) stockant les modèles statistiques, un processeur (102) programmé pour effectuer les étapes de comparaison et de calcul et une sortie pour émettre les notifications d'alerte et de changement de statut.

15. Appareil selon la revendication 14, dans lequel la sortie comprend un affichage (105) destiné à afficher les notifications, l'affichage (105) étant conçu pour afficher les signes vitaux mesurés et le premier et/ou le deuxième indice numérique.

# Fig. 1

# Fig. 2

## Fig. 3(a)

## Fig. 3(b)

## Fig. 3(c)

## Fig. 3(d)

## Fig. 3(e)

## Fig. 3(f)

# Fig. 4

# Fig. 5

Fig. 6

# Fig. 7(a)

# Fig. 7(b)

# Fig. 7(c)

# Fig. 7(d)

# Fig. 7(e)

# Fig. 7(f)

## Fig. 8

## Fig. 9

# Fig. 10

Fig. 11

EOG 1

16.0

mV

14.8

14:16:21.7                                      14:16:26.7

EOG 2

15.7

mV

14.7

14:16:21.7                                      14:16:26.7

EOG 3

15.0

mV

14.5

14:16:21.7                                      14:16:26.7

Oxygen Saturation

14:16:21.7                                      14:16:26.7

Respiration

16950

RPM

16930

14:16:21.7                                      14:16:26.7

Heart Rate                          BPM

# 82

Blood Pressure                    mmHg

# 147₆₇

Oxygen Saturation          % Max

# 97 |

Temperature                          °C

# 35.9

Respiration                          RPM

|

EP 3 142 547 B1

13

# Fig. 12

Observation $y_t$
(HR, BP, BR, SPO2, Temp) — 120

Preprocess - E.G. Normalise
and Decorrelate — 122

Assume that the Observation is
Noise Free and Set $x_t = y_t$ — 124

Read-Off $p(x_t)$ from Eq (1) — 125

Insert $x_t$ into Eq (10) to Calculate
$VSI_{Pop}$ — 126

Alert if $VSI_{Pop}$ (or Filtered Version)
>Threshold — 128

# Fig. 13

130 — Observation $y_t$ (HR, BP, BR, SPO2, Temp)

132 — Preprocess - E.G. Normalise and Decorrelate

134 — Read-Off $p(x_t|y_t)$ from Eq 5

Approach 2 → Insert $p(x_t|y_t)$ into Eq 17 to Calculate VSI$_{Patient}$ — 135

136 — Alert if VSI$_{Patient}$ Beyond Threshold

Approach 1

137 — Insert $p(x_t|y_t)$ into Eq 11 to Calculate VSI$_{Patient}$

Take VSI$_{pop}$ — 138

140 — Status Change Notification

Alert if VSI$_{Pop}$ Increasing and VSI$_{Patient}$ Beyond Threshold — 139

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7031857 B2
- US 20120095520 A1
- US 20080154513 A1
- US 20110068929 A1
- WO 2012160350 A1
- EP 1389948 B1

### Non-patent literature cited in the description

- **J GARDNER-THORPE ; N LOVE ; J WRIGHTSON ; S WALSH ; N KEELING.** The value of modified early warning score (MEWS) in surgical in-patients: a prospective observational study. *Ann. R. Coll. Surg. Engl.,* October 2006, vol. 88 (6), 571-575
- **DAVID A. CLIFTON et al.** Patient-Specific Biomedical Condition Monitoring in Post-operative Cancer Patients. *The sixth International Conference on Condition Monitoring and Machinery Failure Prevention Technology,* 2009, 424-433
- Patient-specific learning in real time for adaptive monitoring in critical care. **Y ZHANG ; P SZOLOVITZ.** Journal of Biomedical Informatics. Academic Press, June 2008, vol. 41, 452-460
- **BRANKO RISTIC.** Beyond the Kalman Filter: Particle Filters for Tracking Applications. Artech House Publishers, 2004
- Adaptive Systems for Signal Processing, Communications, and Control Symposium. **WAN E.A ; VAN DER MERWE, R.** AS-SPCC. IEEE, 2000
- **ARULAMPALAM, M.S. ; MASKELL, S. ; GORDON, N. ; CLAPP, T.** A tutorial on particle filters for online nonlinear/non-Gaussian Bayesian tracking. *IEEE Transactions on Signal Processing,* February 2002, vol. 50 (2), 174, , 188
- **ARULAMPALAM, M.S. ; MASKELL, S. ; GORDON, N. ; CLAPP, T.** A tutorial on particle filters for online nonlinear/non-Gaussian Bayesian tracking. *IEEE Transactions on Signal Processing,* February 2002, vol. 50 (2), 174, , 188